# EUROPEAN PATENT APPLICATION

(11) **EP 4 215 623 A1**
(43) Date of publication of application: **26.07.2023**
(21) Application number: 22152797.1
(22) Date of filing: 21.01.2022
(51) Int. Cl.: C12Q 1/6888

(54) **METHOD FOR MONITORING STRESS IN A SAMPLE OF A FISH AND/OR A FISH CULTURE**

(71) Applicant: Fraunhofer-Gesellschaft zur Förderung der angewandten Forschung e.V., 80686 München (DE)
(72) Inventor: Kruse, Charli, 80686 Munich (DE); Sczakiel, Georg, 80686 Munich (DE)
(74) Representative: Kuttenkeuler, David

(57) **Abstract**

The invention is based on the detection of stress markers in a sample of fish and/or a fish culture, such as an aquaculture water sample. Specifically, the invention provides a method for monitoring fish stress and evaluating fish health in an automated manner. In one aspect, the invention pertains to a method of identifying, predicting, monitoring, and/or diagnosing a stress in a fish and/or a fish culture, wherein a differential level of at least one nucleic acid in the sample compared to a reference sample or reference value or a time-dependent change of the at least one nucleic acid in the sample is indicative for the stress in the fish and/or the fish culture. Further provided is a method of stratifying a fish and/or a fish culture for a culture condition, and a kit for performing a method according to present invention. The invention also pertains to the use of at least one nucleic acid as an indicator for a stress in a fish and/or a fish culture.

## Description

### FIELD OF THE INVENTION

The invention is based on the detection of stress markers in a sample of fish and/or a fish culture, such as an aquaculture water sample. Specifically, the invention provides a method for monitoring fish stress and evaluating fish health in an automated manner. In one aspect, the invention pertains to a method of identifying, predicting, monitoring, and/or diagnosing a stress in a fish and/or a fish culture, wherein a differential level of at least one nucleic acid in the sample compared to a reference sample or reference value or a time-dependent change of the at least one nucleic acid in the sample is indicative for the stress in the fish and/or the fish culture. Further provided is a method of stratifying a fish and/or a fish culture for a culture condition, and a kit for performing a method according to present invention. The invention also pertains to the use of at least one nucleic acid as an indicator for a stress in a fish and/or a fish culture.

### DESCRIPTION

Aquaculture is one of the fastest growing animal production sectors worldwide, and fish in aquaculture are increasingly being used as a substitute for wild-caught fish. Importantly, relocation of fish farming in seas and lakes towards controllable aquaculture in closed containers has the potential to provide a food source for protein-rich animal nutrients in a highly sustainable manner. Fish farming in form of aquaculture can assist in reducing the pollution of the environment, and in providing controllability and an automation of technical processes beneficial for fish farming. Therefore, relocation of aquaculture in seas and lakes towards controllable aquaculture in closed containers is of utmost importance.

It is very common that a high density of fish is often maintained in a single aquaculture container to increase production efficiency of aquaculture. However, a high density of fish in a single container can lead to a drop of water quality due to the accumulation of residual bait and excrement, which can induce stress in fish. Unfortunately, such stress in fish can then induce an increased glucocorticoid level, which lowers immunity of fish and renders the fish more susceptible to diseases. Subsequently, such diseases in fish, for example infectious diseases, could lead to a huge loss of the aquaculture production, i.e. a largely reduced yield. Stress in fish also influences growth, reproduction, and the fitness of fish.

Such stress in fish can be a quasi-social stress, a physical stress, a health stress, a stress caused by an infection or an environmental condition. Moreover, the stress can be characterized by at least one of an altered well-being of the fish, an altered aggressiveness of the fish, a lack of appetite of the fish, a reduced food intake of the fish, and an enhanced susceptibility of the fish for diseases, such as an enhanced susceptibility to a fungal infestation or a worm attack. Exposing fish to any of such kinds of stress can lead to a reduction of the well-being of the cultured fish, which can cause a reduction of the yield of farmed fish and reduce profitability of aquaculture.

Therefore, it is of utmost importance to enable the detection of a modification of the behaviour of cultured fish, and other parameters influencing the growth and well-being of cultured fish. There is also a high demand for novel approaches for the assessment of fish health and fish stress, which is important to secure future global food security.

Current techniques for the assessment of fish stress include the manual observation of an altered behaviour of fish by observant fish farmers. However, this method is very time-consuming and labour-intensive.

Wu et al. (2017) describe an enzyme-functionalized label-free immunosensor system for detecting fish cortisol levels indicative for responses to stress (Wu, Haiyun et al. "New approach for monitoring fish stress: A novel enzyme-functionalized label-free immunosensor system for detecting cortisol levels in fish." Biosensors & bioelectronics 93 (2017): 57-64).

Carbajal et al. (2018) focused on developing and biochemically validating a method for detecting cortisol deposited inside the scales of the goldfish Carassius auratus, and analyzed the suitability of an enzyme immunoassay (EIA) in the quantification of cortisol concentrations in fish scales (Carbajal, A., et al. Cortisol detection in fish scales by enzyme immunoassay: Biochemical and methodological validation. Journal of Applied Ichthyology, 34 (2018): 967-970).

Endo et al. (2019) disclose various biosensors that can both rapidly and easily measure indicators of fish health, which take advantage of molecular elements of biological processes, such as enzymes and antibodies to identify target substances. (Endo, H., Wu, H. Biosensors for the assessment of fish health: a review. Fish Sci 85 (2019): 641-654).

Thus, it is an aim of the present invention to provide standardized and/or automated procedures for the detection of stress markers in a sample of fish and/or a fish culture, such as an aquaculture water sample. It is a further object of the present invention to provide a method that allows to monitor stress in fish to enable subsequent mitigation of stress levels in fish in order to enhance the well-being of fish. Optimising the stress resilience of fish is of utmost importance to make aquaculture more sustainable, and providing a reliable, easy-to-use and fast method for evaluating fish welfare in aquaculture will allow to optimize aquaculture conditions, resulting in a higher yield and healthier high quality fish for the consumer.

### BRIEF DESCRIPTION OF THE INVENTION

Generally, and by way of brief description, the main aspects of the present invention can be described as follows:

In **a first aspect,** the invention pertains to a method of identifying, predicting, monitoring, and/or diagnosing a stress in a fish and/or a fish culture.

In **a second aspect,** the invention pertains to a method of stratifying a fish and/or a fish culture for a culture condition.

In **a third aspect,** the invention pertains to the use of at least one nucleic acid as an indicator for a stress in a fish and/or a fish culture.

In **a fourth aspect,** the invention pertains to a kit for performing a method according to the first or second aspect of the present invention.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the invention pertains to a method of identifying, predicting, monitoring, and/or diagnosing a stress in a fish and/or a fish culture comprising the steps of:
(a) Optionally, providing a sample of the fish and/or the fish culture;
(b) Isolating and/or detecting at least one nucleic acid from the sample of the fish and/or the fish culture;
(c) Determining and/or quantifying the level of the at least one nucleic acid in the sample, and
(d) Comparing the level of the at least one nucleic acid in the sample to a reference sample or reference value, or measuring a time-dependent change of the at least one nucleic acid in the sample,
thereby identifying, predicting, monitoring, and/or diagnosing the stress in the fish and/or the fish culture, wherein a differential level of the at least one nucleic acid in the sample compared to said reference sample or reference value or said time-dependent change of the at least one nucleic acid in the sample is indicative for the stress in the fish and/or the fish culture.

In the course of the present invention, the inventors observed that altered environmental conditions can lead to genetically anchored reactions in living organisms, such as in fish. This causes an altered expression of genes, which are specific indicators of such altered environmental conditions. Interestingly, altered gene expression in living organisms induced by altered environmental conditions can be detected in form of suppressed or enhanced expression of marker genes, for example in form of altered levels of RNA transcripts and/or altered protein levels. This invention now provides a method to isolate and quantitatively detect the expression of marker genes, for example in form of RNA transcripts, in a sample of fish and/or a fish culture. This invention therefore allows monitoring and subsequent mitigation of stress levels in fish in order to enhance the well-being of fish. Thus, the level of the at least one nucleic acid in the sample, such as the level of an RNA polynucleotide molecule, can be used as biomarker for stress quantification in fish.

According to the present invention, the level of the at least one nucleic acid in the sample, such as the level of an RNA polynucleotide molecule, can be determined in any sample of the fish and/or the fish culture. According to one preferred embodiment, the sample is a water sample, such as an aquaculture water sample, a tissue sample or a body liquid sample derived from the fish and/or the fish culture.

Importantly, an automated method allowing to monitor stress markers in fish and/or a fish culture enables to detect stress markers in the sample derived from the fish and/or the fish culture, such as in the aquaculture water sample, in time to initiate countermeasures. This is of utmost importance for enhancing the well-being of fish, and, thereby, enhancing the yield of aquaculture fish farming.

According to the present invention, comparing the level of the at least one nucleic acid in the sample to a reference sample or reference value in step d) of the method is not indispensable for the present invention. Instead, it is also possible to measure a time-dependent change of the at least one nucleic acid in the sample in step d) of the method. For example, a fast and/or substantial change of the stress marker value can also indicate fish stress. Thus, it can be sufficient to solely measure the stress marker without comparing it to a reference sample or reference value.

In a further preferred embodiment, the at least one nucleic acid is a functional or non-functional RNA polynucleotide molecule or a functional or non-functional DNA polynucleotide molecule, such as a single-stranded or doubled-stranded RNA polynucleotide molecule or DNA polynucleotide molecule, or a fragment or derivative thereof, for example an mRNA molecule, an RNA mimic, an RNA precursor, an RNA analogue, an RNA antisense molecule, an inhibitory RNA molecule, a ribozyme, an RNA antisense expression molecule, an RNA interference (RNAi) molecule, an siRNA molecule, an esiRNA molecule, an shRNA molecule, a miRNA molecule, a DNA mimic, a DNA precursor, a DNA analogue, an antisense DNA, a DNA aptamer, a decoy molecule, a GapmeR, a PNA (peptide nucleic acid) molecule, an LNA molecule (locked nucleic acid), a genetic construct for targeted gene editing, such as a CRISPR/Cas₉ construct, a guide nucleic acid (gRNA or gDNA), and/or a tracrRNA, preferably an RNA polynucleotide molecule.

Importantly, measuring RNA polynucleotide molecules as markers for fish stress are of advantage, because RNA molecules are the immediate result of an altered gene expression, such as an altered gene expression induced by altered environmental conditions like stress in fish and/or a fish culture. Thus, changes of RNA molecule markers can be observed prior observing an alteration in protein and/or hormone levels. The method of the present invention therefore allows to detect fish stress even before other molecules, such as proteins and/or hormones, like cortisol, are formed. Such monitoring of RNA molecule stress markers allows the subsequent mitigation of stress levels in fish in order to enhance the well-being of fish, which reduces the cost of fish farming and allows to provide a more reliable yield of farmed fish.

In one embodiment, the at least one nucleic acid comprises at least one modification, for example a chemical modification selected from a modified internucleoside linkage, a modified nucleobase, or a modified sugar moiety, such as a 2'-O-alkyl modification, for example a 2'-O-methoxy-ethyl (MOE) or 2'-O-Methyl (OMe) modification, an ethylene-bridged nucleic acid (ENA), a 2'-fluoro (2'-F) nucleic acid, such as 2'-fluoro N₃-P₅'-phosphoramidite, a 1',5'-anhydrohexitol nucleic acid (HNA), or a locked nucleic acid (LNA).

Any method known to the person of skill can be used for isolating and/or detecting the level of the at least one nucleic acid in the sample in step (b) of the method of the present invention. In one embodiment, the isolating and/or detecting in step (b) involves at least one of:
(i) Isolation of RNA;
(ii) Preparation of RNA, and
(iii) Synthesis of cDNA.

Such a method for isolating and/or detecting the level of the at least one nucleic acid in the sample in step (b) of the method of the present invention can involve the use of a reverse transcriptase, such as M-MuLV reverse transcriptase, and/or an RNase Inhibitor, such as a Thermostabile Enzym RiboLock RNase Inhibitor.

In one particularly preferred embodiment of the present invention, the isolating and/or detecting in step (b) of the method is performed by an RNA detection device.

In one embodiment, the level of said at least one nucleic acid is determined and/or quantified in step (c) by means of a detection method comprising mRNA analysis, DNA analysis, and/or protein analysis, such as a method selected from polymerase chain reaction (PCR), real-time quantitative PCR (qPCR), nanoString nCounter, enzyme-linked immunosorbent assay (ELISA), mass spectrometry, mass spectrometry immunoassay (MSIA), antibody-based protein chips, 2-dimensional gel electrophoresis, stable isotope standard capture with anti-peptide antibodies (SISCAPA), high-performance liquid chromatography (HPLC), genetic testing, western blot, cytometry bead array (CBA), radio immunoassay, or immunohistochemical staining, preferably wherein said detection method is real-time quantitative PCR (qPCR).

A further embodiment pertains to the method of the first aspect of the present invention, wherein the level of said at least one nucleic acid is determined and/or quantified in step (c) by means of a measuring sensor, such as a measuring sensor for continuous sampling of the sample of the fish and/or the fish culture sample. According to the present invention, the measuring sensor can use different methods and detect different parameters for measuring and continuously sampling the water of the fish and/or the fish culture.

Importantly, an automated method to monitor stress markers in fish and/or a fish culture enables to detect stress markers in the sample derived from the fish and/or the fish culture, such as in the aquaculture water sample, in time to initiate countermeasures. This is important to enhance the well-being of fish, and, thereby, reduces the cost of fish farming and allows to provide a more reliable yield of farmed fish.

According to the present invention, any kind of stress can be analysed in fish and/or a fish culture. In one embodiment, the stress is a quasi-social stress, a physical stress, a health stress, a stress caused by an infection and/or an environmental condition, optionally wherein the stress is characterized by at least one of an altered well-being of the fish and/or the fish culture, an altered aggressiveness of the fish and/or the fish culture, a lack of appetite of the fish and/or the fish culture, a reduced food intake of the fish and/or the fish culture, and an enhanced susceptibility of the fish and/or the fish culture for a disease, such as an enhanced susceptibility for a fungal infestation or a worm attack. In a further embodiment, the stress is characterized by a stocking density, a water quality, a system design and handling processes such as grading, sampling and transportation. As such, a high stocking density, a poor water quality, a poor system design and/or poor handling processes can induce stress in fish.

The present invention can be used to detect stress in any kind of fish. In one embodiment, the fish and/or the fish culture is selected from common salmon or Atlantic salmon (Salmo salar), barramundi (Lates calcarifer), carp cabezona (Hypophthalmichthys nobilis), tunas (Thunnus), catla (Catla catla), American catfish (lctalurus punctatus), cichlids (Cichlidae), cobia or cod (Rachycentron canadum), Pacific salmon (Oncorhynchus kisutch), common carp European (Cyprinus carpió), cyprinids (Cyprinidae), eel or common eel (Anguilla anguilla), bass (Dicentrarchus labrax), mullet (Mugil cephalus), gilt (Sparus aurata), herbivorous carp (Ctenopharyngodon idellus), Japanese eel (Japan eel), mandarin fish (Siniperca chuatsi), regal perch (Argyrosomus regius), sabalote or milk fish (Chanos Chanos), mrigal carp (Cirrhinus mhgala), Nile tilapia (Oreochromis niloticus), Pangasius, Plagioscion, branch (Trachinotus), rainbow trout (Oncorhynchus mykiss), red drum (Sciaenops ocellatus), labeo roho (Labeo rohita), lemon fish (Seriola), Siberian sturgeon (Acipenser baerii), silver carp (Hypophthalrixthysmichthys), striped catfish (Pangasius hypophthalmus), turbot (Psetta maxim), snakehead fish (Channa argus), yellowfin tuna (Thunnus albacares), lobster (Palinurus), shrimp (Caridea), crab (Brachyura infraorder), prawns (infraorder Dendrobranchiata) and barnacles (Pollicipes).

In a particularly preferred embodiment, the method is an ex-vivo, in-vivo, or in-vitro method, preferably a non-invasive method, such as an in-vitro method.

In another embodiment, said at least one nucleic acid is selected from 18S rRNA and elongation factor 1 alpha (EF1α). Interestingly, in the course of the present invention, the inventors observed that 18S rRNA and EF1α substantially increase upon the transient induction of stress in fish.

In **the second aspect,** the invention pertains to a method of stratifying a fish and/or a fish culture for a culture condition, said method comprising, as a first step, identifying a differential level of at least one nucleic acid in a sample of the fish and/or the fish culture compared to a reference sample or reference value or a time-dependent change of the at least one nucleic acid in the sample and, as a second step, deciding in favor of or against altering said culture condition, wherein said differential level of the at least one nucleic acid is identified by using a method comprising the steps
(a) Optionally, providing a sample of the fish and/or the fish culture;
(b) Isolating and/or detecting at least one nucleic acid from the sample of the fish and/or the fish culture;
(c) Determining and/or quantifying the level of the at least one nucleic acid in the sample, and
(d) Comparing the level of the at least one nucleic acid in the sample to a control level or a reference sample or reference value, or measuring the time-dependent change of the at least one nucleic acid in the sample,
wherein if a differential level of the at least one nucleic acid in the sample compared to said reference sample or reference value or if said time-dependent change of the at least one nucleic acid in the sample is detected, said culture condition is altered.

According to one preferred embodiment of the second aspect of the present invention, the sample is a water sample, such as an aquaculture water sample, a tissue sample or a body liquid sample derived from the fish and/or the fish culture.

In a further preferred embodiment of the second aspect of the present invention, the at least one nucleic acid is a functional or non-functional RNA polynucleotide molecule or a functional or non-functional DNA polynucleotide molecule, such as a single-stranded or doubled-stranded RNA polynucleotide molecule or DNA polynucleotide molecule, or a fragment or derivative thereof, for example an mRNA molecule, an RNA mimic, an RNA precursor, an RNA analogue, an RNA antisense molecule, an inhibitory RNA molecule, a ribozyme, an RNA antisense expression molecule, an RNA interference (RNAi) molecule, an siRNA molecule, an esiRNA molecule, an shRNA molecule, a miRNA molecule, a DNA mimic, a DNA precursor, a DNA analogue, an antisense DNA, a DNA aptamer, a decoy molecule, a GapmeR, a PNA (peptide nucleic acid) molecule, an LNA molecule (locked nucleic acid), a genetic construct for targeted gene editing, such as a CRISPR/Cas₉ construct, a guide nucleic acid (gRNA or gDNA), and/or a tracrRNA, preferably an RNA polynucleotide molecule.

In one embodiment of the second aspect of the present invention, the at least one nucleic acid comprises at least one modification, for example a chemical modification selected from a modified internucleoside linkage, a modified nucleobase, or a modified sugar moiety, such as a 2'-O-alkyl modification, for example a 2'-O-methoxy-ethyl (MOE) or 2'-O-Methyl (OMe) modification, an ethylene-bridged nucleic acid (ENA), a 2'-fluoro (2'-F) nucleic acid, such as 2'-fluoro N₃-P₅'-phosphoramidite, a 1',5'-anhydrohexitol nucleic acid (HNA), or a locked nucleic acid (LNA).

Any method known to the person of skill can be used for isolating and/or detecting the level of the at least one nucleic acid in the sample in step (b) of the method of the second aspect of the present invention. In one embodiment, the isolating and/or detecting in step (b) involves at least one of:
(iv) Isolation of RNA;
(v) Preparation of RNA, and
(vi) Synthesis of cDNA.

Such a method for isolating and/or detecting the level of the at least one nucleic acid in the sample in step (b) of the method of the second aspect of the present invention can involve the use of a reverse transcriptase, such as M-MuLV reverse transcriptase, and/or an RNase Inhibitor, such as a Thermostabile Enzym RiboLock RNase Inhibitor.

In one particularly preferred embodiment of the present invention, the isolating and/or detecting in step (b) of the method of the second aspect of the present invention is performed by an RNA detection device.

In one embodiment of the second aspect of the present invention, the level of said at least one nucleic acid is determined and/or quantified in step (c) by means of a detection method comprising mRNA analysis, DNA analysis, and/or protein analysis, such as a method selected from polymerase chain reaction (PCR), real-time quantitative PCR (qPCR), nanoString nCounter, enzyme-linked immunosorbent assay (ELISA), mass spectrometry, mass spectrometry immunoassay (MSIA), antibody-based protein chips, 2-dimensional gel electrophoresis, stable isotope standard capture with anti-peptide antibodies (SISCAPA), high-performance liquid chromatography (HPLC), genetic testing, western blot, cytometry bead array (CBA), radio immunoassay, or immunohistochemical staining, preferably wherein said detection method is real-time quantitative PCR (qPCR).

A further embodiment of the second aspect of the present invention pertains to the method of the present invention, wherein the level of said at least one nucleic acid is determined and/or quantified in step (c) by means of a measuring sensor, such as a measuring sensor for continuous sampling of the sample of the fish and/or the fish culture water sample. According to the present invention, the measuring sensor can use different methods for measuring and continuously sample the water of the fish and/or the fish culture.

According to the present invention, any kind of stress can be analysed in fish and/or a fish culture. In one embodiment, the stress is a quasi-social stress, a physical stress, a health stress, a stress caused by an infection and/or an environmental condition, optionally wherein the stress is characterized by at least one of an altered well-being of the fish and/or the fish culture, an altered aggressiveness of the fish and/or the fish culture, a lack of appetite of the fish and/or the fish culture, a reduced food intake of the fish and/or the fish culture, and an enhanced susceptibility of the fish and/or the fish culture for a disease, such as an enhanced susceptibility for a fungal infestation or a worm attack. In a further embodiment, the stress is characterized by a stocking density, a water quality, a system design and handling processes such as grading, sampling and transportation. As such, a high stocking density, a poor water quality, a poor system design and/or poor handling processes can induce stress in fish.

In another embodiment, the fish and/or the fish culture is selected from common salmon or Atlantic salmon (Salmo salar), barramundi (Lates calcarifer), carp cabezona (Hypophthalmichthys nobilis), tunas (Thunnus), catla (Catla catla), American catfish (lctalurus punctatus), cichlids (Cichlidae), cobia or cod (Rachycentron canadum), Pacific salmon (Oncorhynchus kisutch), common carp European (Cyprinus carpió), cyprinids (Cyprinidae), eel or common eel (Anguilla anguilla), bass (Dicentrarchus labrax), mullet (Mugil cephalus), gilt (Sparus aurata), herbivorous carp (Ctenopharyngodon idellus), Japanese eel (Japan eel), mandarin fish (Siniperca chuatsi), regal perch (Argyrosomus regius), sabalote or milk fish (Chanos Chanos), mrigal carp (Cirrhinus mhgala), Nile tilapia (Oreochromis niloticus), Pangasius, Plagioscion, branch (Trachinotus), rainbow trout (Oncorhynchus mykiss), red drum (Sciaenops ocellatus), labeo roho (Labeo rohita), lemon fish (Seriola), Siberian sturgeon (Acipenser baerii), silver carp (Hypophthalrixthysmichthys), striped catfish (Pangasius hypophthalmus), turbot (Psetta maxim), snakehead fish (Channa argus), yellowfin tuna (Thunnus albacares), lobster (Palinurus), shrimp (Caridea), crab (Brachyura infraorder), prawns (infraorder Dendrobranchiatá) and barnacles (Pollicipes).

In a particularly preferred embodiment, the method of the second aspect of the present invention is an ex-vivo, in-vivo, or in-vitro method, preferably a non-invasive method, such as an in-vitro method.

In another embodiment, said at least one nucleic acid is selected from 18S rRNA and elongation factor 1 alpha (EF1α).

In **the third aspect,** the invention pertains to the use of at least one nucleic acid as an indicator for a stress in a fish and/or a fish culture.

In a preferred embodiment of the third aspect of the present invention, the at least one nucleic acid is a functional or non-functional RNA polynucleotide molecule or a functional or non-functional DNA polynucleotide molecule, such as a single-stranded or doubled-stranded RNA polynucleotide molecule or DNA polynucleotide molecule, or a fragment or derivative thereof, for example an mRNA molecule, an RNA mimic, an RNA precursor, an RNA analogue, an RNA antisense molecule, an inhibitory RNA molecule, a ribozyme, an RNA antisense expression molecule, an RNA interference (RNAi) molecule, an siRNA molecule, an esiRNA molecule, an shRNA molecule, a miRNA molecule, a DNA mimic, a DNA precursor, a DNA analogue, an antisense DNA, a DNA aptamer, a decoy molecule, a GapmeR, a PNA (peptide nucleic acid) molecule, an LNA molecule (locked nucleic acid), a genetic construct for targeted gene editing, such as a CRISPR/Cas₉ construct, a guide nucleic acid (gRNA or gDNA), and/or a tracrRNA, preferably an RNA polynucleotide molecule.

In another embodiment of the third aspect of the present invention, the at least one nucleic acid comprises at least one modification, for example a chemical modification selected from a modified internucleoside linkage, a modified nucleobase, or a modified sugar moiety, such as a 2'-O-alkyl modification, for example a 2'-O-methoxy-ethyl (MOE) or 2'-O-Methyl (OMe) modification, an ethylene-bridged nucleic acid (ENA), a 2'-fluoro (2'-F) nucleic acid, such as 2'-fluoro N₃-P₅'-phosphoramidite, a 1',5'-anhydrohexitol nucleic acid (HNA), or a locked nucleic acid (LNA).

According to the present invention, any kind of stress can be analysed in fish and/or a fish culture. In one embodiment of the third aspect of the present invention, the stress is a quasi-social stress, a physical stress, a health stress, a stress caused by an infection and/or an environmental condition, optionally wherein the stress is characterized by at least one of an altered well-being of the fish and/or the fish culture, an altered aggressiveness of the fish and/or the fish culture, a lack of appetite of the fish and/or the fish culture, a reduced food intake of the fish and/or the fish culture, and an enhanced susceptibility of the fish and/or the fish culture for a disease, such as an enhanced susceptibility for a fungal infestation or a worm attack. In a further embodiment, the stress is characterized by a stocking density, a water quality, a system design and handling processes such as grading, sampling and transportation. As such, a high stocking density, a poor water quality, a poor system design and/or poor handling processes can induce stress in fish.

In another embodiment, the fish and/or the fish culture is selected from common salmon or Atlantic salmon (Salmo salar), barramundi (Lates calcarifer), carp cabezona (Hypophthalmichthys nobilis), tunas (Thunnus), catla (Catla catla), American catfish (lctalurus punctatus), cichlids (Cichlidae), cobia or cod (Rachycentron canadum), Pacific salmon (Oncorhynchus kisutch), common carp European (Cyprinus carpió), cyprinids (Cyprinidae), eel or common eel (Anguilla anguilla), bass (Dicentrarchus labrax), mullet (Mugil cephalus), gilt (Sparus aurata), herbivorous carp (Ctenopharyngodon idellus), Japanese eel (Japan eel), mandarin fish (Siniperca chuatsi), regal perch (Argyrosomus regius), sabalote or milk fish (Chanos Chanos), mrigal carp (Cirrhinus mhgala), Nile tilapia (Oreochromis niloticus), Pangasius, Plagioscion, branch (Trachinotus), rainbow trout (Oncorhynchus mykiss), red drum (Sciaenops ocellatus), labeo roho (Labeo rohita), lemon fish (Seriola), Siberian sturgeon (Acipenser baerii), silver carp (Hypophthalrixthysmichthys), striped catfish (Pangasius hypophthalmus), turbot (Psetta maxim), snakehead fish (Channa argus), yellowfin tuna (Thunnus albacares), lobster (Palinurus), shrimp (Caridea), crab (Brachyura infraorder), prawns (infraorder Dendrobranchiatá) and barnacles (Pollicipes).

In another embodiment, said at least one nucleic acid is selected from 18S rRNA and elongation factor 1 alpha (EF1α).

In **the fourth aspect,** the invention pertains to a kit for performing a method according to the first or second aspect of the present invention, the kit comprising means for isolating and/or detecting at least one nucleic acid from a sample, and/or means for determining and/or quantifying the level of the at least one nucleic acid in the sample.

In a preferred embodiment of the fourth aspect of the present invention, the at least one nucleic acid is a functional or non-functional RNA polynucleotide molecule or a functional or non-functional DNA polynucleotide molecule, such as a single-stranded or doubled-stranded RNA polynucleotide molecule or DNA polynucleotide molecule, or a fragment or derivative thereof, for example an mRNA molecule, an RNA mimic, an RNA precursor, an RNA analogue, an RNA antisense molecule, an inhibitory RNA molecule, a ribozyme, an RNA antisense expression molecule, an RNA interference (RNAi) molecule, an siRNA molecule, an esiRNA molecule, an shRNA molecule, a miRNA molecule, a DNA mimic, a DNA precursor, a DNA analogue, an antisense DNA, a DNA aptamer, a decoy molecule, a GapmeR, a PNA (peptide nucleic acid) molecule, an LNA molecule (locked nucleic acid), a genetic construct for targeted gene editing, such as a CRISPR/Cas₉ construct, a guide nucleic acid (gRNA or gDNA), and/or a tracrRNA, preferably an RNA polynucleotide molecule.

In another embodiment of the fourth aspect of the present invention, the at least one nucleic acid comprises at least one modification, for example a chemical modification selected from a modified internucleoside linkage, a modified nucleobase, or a modified sugar moiety, such as a 2'-O-alkyl modification, for example a 2'-O-methoxy-ethyl (MOE) or 2'-O-Methyl (OMe) modification, an ethylene-bridged nucleic acid (ENA), a 2'-fluoro (2'-F) nucleic acid, such as 2'-fluoro N₃-P₅'-phosphoramidite, a 1',5'-anhydrohexitol nucleic acid (HNA), or a locked nucleic acid (LNA).

In one embodiment, the kit comprises means for isolating and/or detecting at least one nucleic acid selected from 18S rRNA and elongation factor 1 alpha (EF1α).

According to another preferred embodiment of the fourth aspect of the present invention, the sample is a water sample, such as an aquaculture water sample, a tissue sample or a body liquid sample derived from the fish and/or the fish culture.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

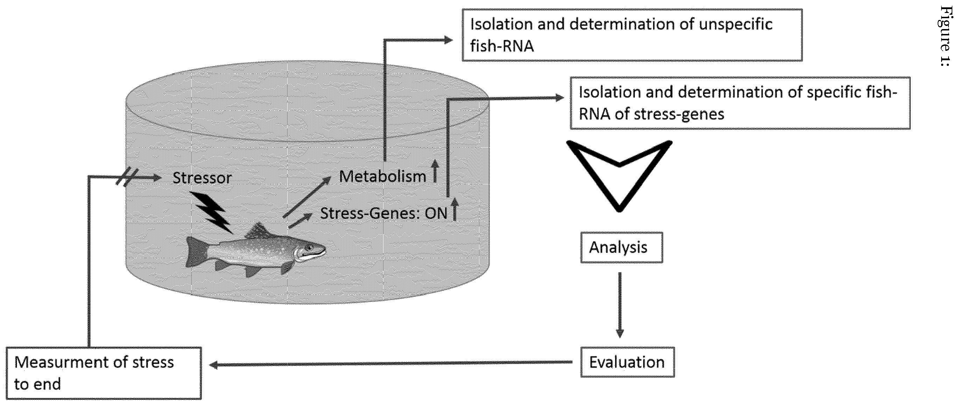

**Figure 1** shows a schematic representation of the invention described. Fish or fish cultures are exposed to a stressor. This leads to a change in the metabolism and/or to the activation of stress genes. Unspecific and stress-specific fish RNAs are isolated from the fish or fish culture (e.g. aquaculture water) and then determined, analyzed and evaluated. If the evaluation is positive, the stressor in the culture system is removed and the fish's welfare is restored.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The example show:

### Example 1: Experimental detection of RNA

**RNA isolation from water:** Total RNA from the water samples was isolated using QIAgen's RNeasy Midi Column Kit, based on the column separation method. This technology combines the selective binding properties of the silica gel-based membrane with centrifugation. A specialized high salt buffer system allows RNAs longer than 200 bp to be bound to the membrane, washed and eluted.

The addition of 250 µL HEPES pH 7.0 maintains the pH in the physiological range. As an internal control, 8 µL of 1.25 x 10⁶ C/µL luciferase RNA was added, which corresponds to a total copy number of 1 x 10⁷ of luciferase RNA. 2.5 mL of 70 % ethanol was added to provide optimal binding conditions. The addition of 25 µL of beta-mercapthoethanol ensured denaturation of the proteins as the disulfid bridges were reduced.

4 mL of the prepared water samples were pipetted onto the column standing in a centrifuge tube and centrifuged at 4,000 x g for 5 min. The centrifugate was decanted. To remove all unbound molecules in a wash step, 2 mL wash buffer RW1 was added to the column and centrifuged again at 4,000 x g for 5 min.

DNase digestion with the help of the enzyme DNase I takes place on the membrane. The enzyme DNase I is a specific endonuclease and degrades single- and double-stranded DNA to short oligonucleotides with 5'phosphate group and 3'OH group. For DNA hydrolysis, 55 U DNase I in RDD buffer was added directly to the membrane and incubated for 5 min. at room temperature (RT). The master mix for one batch consisted of 20 µL DNase 1 and 140 µL RDD buffer. After the incubation period, 2 mL of RW1 buffer was added, incubated for 5 min. at RT and centrifuged at 4,000 x g for 5 min. Washing of the RNA with 2.5 mL RPE buffer followed by centrifugation for 2 min. at 4,000 x g was repeated twice. Then, the centrifugate was decanted. After another centrifugation step for 5 min. at 4,000 x g, the column was transferred to a new centrifuge tube.

The elution of the RNA was carried out twice with the addition of 160 µL RNase-free water, an incubation time of 1 min. and 3 min. centrifugation at 4,000 x g. The total RNA was in a volume of 360 µL and was frozen in liquid nitrogen to be stored at -80 °C.

The obtained RNA solution was lyophilized under low drying rate to increase the concentration. The procedure was carried out until no more liquid was present and the RNA was in the form of a pellet. The resulting pellet was resuspended on ice in 16 µL RNase-free water.

**Subsequent steps** - **cDNA synthesis:** In order to carry out an analysis by qPCR, a DNA synthesis of the RNA with random hexamer primer had to be carried out by a reverse transcriptase. The random hexamer primer used was a mixture of single-stranded hexanucleotides that bound to randomly complementary sites of a target DNA, thus allowing the largest possible reservoir of DNA to be synthesized.

For the cDNA synthesis of tissue RNA as well as water RNA, the RevertAid First Stand cDNA Synthesis Kit (ThermoFisher Scienfic) was used. The kit contained a recombinant M-MuLV reverse transcriptase (RT), which synthesizes DNA up to a length of 13 kb, and the thermostable enzyme RiboLock RNase Inhibitor, which inhibits RNase A, B and C. Two different master mixes of RT were used. Two different master mixes with RT and without RT (NoRT) were chosen. The first mix RT contained the RT as well as the RNase inhibitor, so that the RNA could be successfully transcribed into DNA. In the second mix NoRT, however, the enzyme and the inhibitor were missing, so that no DNA could be synthesized. These approaches served as controls.

**Analysis of RNA:** The absorbance of nucleic acids is typically measured photometrically at 260 nm and used for concentration determinations. For the purity determination of nucleic acids, the 260/280 nm ratio is used. At a ratio of 2.0 DNA is considered pure, RNA at a ratio of 1.8. Lower ratios of the 260/280 nm ratio can be an indication of impurities with substances such as proteins, which absorb at or near 280 nm. In addition, the 230/260 nm ratio can be used for purity testing, which for nucleic acids is between 2.0 and 2.2. The presence of contaminants such as phenols may be responsible for lowering the value. The molarities were calculated from the absorbance using Lambert-Beer's law.

**PCR amplification and sequencing of the products:** The goal of sequencing the PCR products using the BigDye Terminator v3.1 Sequencing Kit was to confirm that EFia had indeed been amplified. In the Sanger chain termination method, the DNA to be sequenced was incubated by a polymerase with suitable primers and dNTPs as well as ddNTPs. The random insertion of ddNTPs resulted in a chain termination, as no further phosphodiester bonds could be linked due to the missing 3'OH group. In addition, each ddNTP was labelled with a different fluorescent dye. The DNA sequences of different lengths, which are separated according to their length with the aid of capillary electrophoresis and detected by a detector at their labelling, are produced by the random termination reaction. The various signals that correlate with the bases are shown in the time course of the electrophoresis.

The sequencing was divided into four sequential steps of sample preparation, sequencing reaction, purification and capillary electrophoresis. The ExoSAP digestion was used to enzymatically purify the amplified PCR product. In the course of purification, 2 µL of ExoSAP was added to 5 µL of PCR product. Then, excess primers were degraded and nucleotides dephosphorylated in a first incubation step for 4 min. at 37 °C. Subsequently, the enzyme was inactivated in a second incubation step for 1 min. at 95 °C. The sequencing set was prepared for the forward primer as well as the reverse primer and incubated according to the PCR manual.

### References

(1) Wu, Haiyun et al. "New approach for monitoring fish stress: A novel enzyme-functionalized label-free immunosensor system for detecting cortisol levels in fish." Biosensors & bioelectronics 93 (2017): 57-64.
(2) Carbajal, A., Monclus, L., Tallo-Parra, O., Sabes-Alsina, M., Vinyoles, D., & Lopez-Bejar, M. (2018). Cortisol detection in fish scales by enzyme immunoassay: Biochemical and methodological validation. Journal of Applied Ichthyology, 34, 967-970. https://doi.org/10.1111/jai.13674.
(3) Endo, H., Wu, H. Biosensors for the assessment of fish health: a review. Fish Sci 85, 641-654 (2019). https://doi.org/10.1007/S12562-019-01318-y.

## Claims

1. A method of identifying, predicting, monitoring, and/or diagnosing a stress in a fish and/or a fish culture comprising the steps of:
(a) Optionally, providing a sample of the fish and/or the fish culture;
(b) Isolating and/or detecting at least one nucleic acid from a sample of the fish and/or the fish culture;
(c) Determining and/or quantifying the level of the at least one nucleic acid in the sample, and
(d) Comparing the level of the at least one nucleic acid in the sample to a reference sample or reference value, or measuring a time-dependent change of the at least one nucleic acid in the sample,
thereby identifying, predicting, monitoring, and/or diagnosing the stress in the fish and/or the fish culture, wherein a differential level of the at least one nucleic acid in the sample compared to said reference sample or reference value or said time-dependent change of the at least one nucleic acid in the sample is indicative for the stress in the fish and/or the fish culture.

2. The method according to claim 1, wherein the sample is a water sample, such as an aquaculture water sample, a tissue sample or a body liquid sample derived from the fish and/or the fish culture.

3. The method according to claim 1 or 2, wherein the at least one nucleic acid is a functional or non-functional RNA polynucleotide molecule or a functional or non-functional DNA polynucleotide molecule, such as a single-stranded or doubled-stranded RNA polynucleotide molecule or DNA polynucleotide molecule, or a fragment or derivative thereof, for example an mRNA molecule, an RNA mimic, an RNA precursor, an RNA analogue, an RNA antisense molecule, an inhibitory RNA molecule, a ribozyme, an RNA antisense expression molecule, an RNA interference (RNAi) molecule, an siRNA molecule, an esiRNA molecule, an shRNA molecule, a miRNA molecule, a DNA mimic, a DNA precursor, a DNA analogue, an antisense DNA, a DNA aptamer, a decoy molecule, a GapmeR, a PNA (peptide nucleic acid) molecule, an LNA molecule (locked nucleic acid), a genetic construct for targeted gene editing, such as a CRISPR/Cas₉ construct, a guide nucleic acid (gRNA or gDNA), and/or a tracrRNA, preferably an RNA polynucleotide molecule.

4. The method according to any one of claims 1 to 3, wherein the at least one nucleic acid comprises at least one modification, for example a chemical modification selected from a modified internucleoside linkage, a modified nucleobase, or a modified sugar moiety, such as a 2'-O-alkyl modification, for example a 2'-O-methoxy-ethyl (MOE) or 2'-O-Methyl (OMe) modification, an ethylene-bridged nucleic acid (ENA), a 2'-fluoro (2'-F) nucleic acid, such as 2'-fluoro N₃-P₅'-phosphoramidite, a 1',5'-anhydrohexitol nucleic acid (HNA), or a locked nucleic acid (LNA).

5. The method according to any one of claims 1 to 4, wherein the isolating and/or detecting in step (b) involves at least one of:
(i) Isolation of RNA;
(ii) Preparation of RNA, and
(iii) Synthesis of cDNA.

6. The method according to any one of claims 1 to 5, wherein the isolating and/or detecting in step (b) is performed by an RNA detection device.

7. The method according to any one of claims 1 to 6, wherein the level of said at least one nucleic acid is determined and/or quantified in step (c) by means of a detection method comprising mRNA analysis, DNA analysis, and/or protein analysis, such as a method selected from polymerase chain reaction (PCR), real-time quantitative PCR (qPCR), nanoString nCounter, enzyme-linked immunosorbent assay (ELISA), mass spectrometry, mass spectrometry immunoassay (MSIA), antibody-based protein chips, 2-dimensional gel electrophoresis, stable isotope standard capture with anti-peptide antibodies (SISCAPA), high-performance liquid chromatography (HPLC), genetic testing, western blot, cytometry bead array (CBA), radio immunoassay, or immunohistochemical staining, preferably wherein said detection method is real-time quantitative PCR (qPCR).

8. The method according to any one of claims 1 to 7, wherein the level of said at least one nucleic acid is determined and/or quantified in step (c) by means of a measuring sensor, such as a measuring sensor for continuous sampling of the sample of the fish and/or the fish culture sample.

9. The method according to any one of claims 1 to 8, wherein the stress is a quasi-social stress, a physical stress, a health stress, a stress caused by an infection and/or an environmental condition, optionally wherein the stress is **characterized by** at least one of an altered well-being of the fish and/or the fish culture, an altered aggressiveness of the fish and/or the fish culture, a lack of appetite of the fish and/or the fish culture, a reduced food intake of the fish and/or the fish culture, and an enhanced susceptibility of the fish and/or the fish culture for a disease, such as an enhanced susceptibility for a fungal infestation or a worm attack.

10. The method according to any one of claims 1 to 9, wherein the fish and/or the fish culture is selected from common salmon or Atlantic salmon (Salmo salar), barramundi (Lates calcarifer), carp cabezona (Hypophthalmichthys nobilis), tunas (Thunnus), catla (Catla catla), American catfish (lctalurus punctatus), cichlids (Cichlidae), cobia or cod (Rachycentron canadum), Pacific salmon (Oncorhynchus kisutch), common carp European (Cyprinus carpió), cyprinids (Cyprinidae), eel or common eel (Anguilla anguilla), bass (Dicentrarchus labrax), mullet (Mugil cephalus), gilt (Sparus aurata), herbivorous carp (Ctenopharyngodon idellus), Japanese eel (Japan eel), mandarin fish (Siniperca chuatsi), regal perch (Argyrosomus regius), sabalote or milk fish (Chanos Chanos), mrigal carp (Cirrhinus mhgala), Nile tilapia (Oreochromis niloticus), Pangasius, Plagioscion, branch (Trachinotus), rainbow trout (Oncorhynchus mykiss), red drum (Sciaenops ocellatus), labeo roho (Labeo rohita), lemon fish (Seriola), Siberian sturgeon (Acipenser baerii), silver carp (Hypophthalrixthysmichthys), striped catfish (Pangasius hypophthalmus), turbot (Psetta maxim), snakehead fish (Channa argus), yellowfin tuna (Thunnus albacares), lobster (Palinurus), shrimp (Caridea), crab (Brachyura infraorder), prawns (infraorder Dendrobranchiatá) and barnacles (Pollicipes).

11. The method according to any one of claims 1 to 10, wherein the method is an *ex-νiνo,* in-*vivo,* or *in-νitro* method, preferably a non-invasive method, such as an *in-νitro* method.

12. The method according to any one of claims 1 to 11, wherein said at least one nucleic acid is selected from 18S rRNA and elongation factor 1 alpha (EF1α).

13. A method of stratifying a fish and/or a fish culture for a culture condition, said method comprising, as a first step, identifying a differential level of at least one nucleic acid in a sample of the fish and/or the fish culture compared to a reference sample or reference value or a time-dependent change of the at least one nucleic acid in the sample and, as a second step, deciding in favor of or against altering said culture condition, wherein said differential level of the at least one nucleic acid is identified by using a method comprising the steps of:
(a) Optionally, providing a sample of the fish and/or the fish culture;
(b) Isolating and/or detecting at least one nucleic acid from a sample of the fish and/or the fish culture;
(c) Determining and/or quantifying the level of the at least one nucleic acid in the sample, and
(d) Comparing the level of the at least one nucleic acid in the sample to a reference sample or reference value, or measuring the time-dependent change of the at least one nucleic acid in the sample,
wherein if a differential level of the at least one nucleic acid in the sample compared to said reference sample or reference value or if a time-dependent change of the at least one nucleic acid in the sample is detected, said culture condition is altered.

14. Use of at least one nucleic acid as an indicator for a stress in a fish and/or a fish culture.

15. A kit for performing a method according to any one of claims 1 to 13, the kit comprising means for isolating and/or detecting at least one nucleic acid from a sample, and/or means for determining and/or quantifying the level of the at least one nucleic acid in the sample.
